# EUROPEAN PATENT APPLICATION

(11) **EP 4 190 894 A1**
(43) Date of publication of application: **07.06.2023**
(21) Application number: 21212360.8
(22) Date of filing: 03.12.2021
(51) Int. Cl.: C12N 5/0784

(54) **METHOD FOR THE PRODUCTION OF MATURE DENDRITIC CELLS IN VITRO USING A BACTERIAL LYSATE**

(71) Applicant: CiMaas B.V., 6229 EV Maastricht (NL)
(72) Inventor: GERMERAAD, Wilfred Thomas Vincent, 6229 EV MAASTRICHT (NL); BOS, Gerardus Marinus Josef, 6229 EV MAASTRICHT (NL)
(74) Representative: Algemeen Octrooi- en Merkenbureau B.V.

(57) **Abstract**

The invention is in the field of cell biology and immunology. The invention provides methods for the production of a vaccine for cancer and infectious diseases. More in particular it provides a method for the improved production of mature dendritic cells. This improved production includes the use of a bacterial lysate produced by a new method.

## Description

### Field of the invention

The invention is in the field of cell biology and immunology. The invention provides methods for the production of a vaccine for cancer and infectious diseases. More in particular it provides a method for the improved production of mature dendritic cells. This improved production includes the use of a bacterial lysate produced by a new method.

### Background of the invention

Despite improvements in conventional cancer treatments, such as surgery, chemo- and radiotherapy, cancer is still a leading cause of death worldwide (1). Current anti-cancer therapies cure about 60% of all patients. However, in metastatic disease of solid tumors and in several stages of hematological malignancies therapeutic options are limited and do not lead to cure (1). In addition, the treatment options might pose serious side-effects. Therefore, there is an urgent need for more effective and less invasive therapies. A growing field of interest in the search for novel therapeutic strategies is that of immunotherapy. As the immune system of many cancer patients is suppressed, the first line of defense against malignant cells is lost. This creates a tumor permissive environment.

Immunotherapy aims to overcome this suppression and reactivate the patient's own immune system to reject the tumor or to make use of donor derived cells. There are various strategies already being used in the clinic, utilizing both active and passive immunity.

The treatment of cancer with monoclonal antibodies is one example of passive immunotherapy. Monoclonal antibodies to known tumor antigens are injected, where they recognize and bind to their specific epitope. These antibodies are used alone, relying on antibody-dependent cell-mediated cytotoxicity or an anti-apoptotic signal, or are coupled to therapeutic agents allowing for localized administration. Monoclonal antibodies have shown positive results on both long-term and short-term survival. The downside of this strategy is that most tumor-associated epitopes are located within the cell, making them inaccessible for antibodies. Additionally, few antigens are tumor-specific resulting in unwanted targeting of healthy cells.

Other examples of passive immunotherapies are immune adjuvants and cytokines. These therapies aim at creating an inflammatory environment that would counteract the immunosuppressive nature of the malignancy, allowing spontaneous immune responses to take place. However, results using these therapies are limited. The downside of passive immunotherapies in general, is that memory immune cells are not formed, making relapse likely.

These disadvantages may be circumvented by active immunotherapy, which initiates an immune response against the tumor, thereby not only making use of the (antigen-specific) effector cells, such as cytotoxic lymphocytes (CTL), plasma cells and NK cells, but also of protective memory. One strategy for active immunotherapy is through vaccination with dendritic cells (DC). These cells are the most important antigen-presenting cells in the body, capable of initiating both innate and adaptive immune responses.

We have developed immunotherapy with dendritic cells (DC) to potently stimulate the patients' own immune system to induce anti-tumor effects. We also developed a DC vaccine that has a strong capacity to in vitro stimulate immune cells as T cells and Natural Killer cells (35; 51-57). This vaccine technology is based on autologous dendritic cells, which are activated using an ex vivo maturation process which can be applied for many cancers (Patent EP 2847321 B1).

In cancer patients, DC have been shown to be impaired in their activity, therefore ex vivo reactivation and education is an attractive approach to apply them in immunotherapy (2). These and other immunotherapies, are extensively reviewed in literature references 3 and 4.

DC-based vaccines are already in use in the clinic, revealing no or only mild side-effects (5). However, only 10 - 15% of the patients thus treated responded to the treatment (5). This indicates that improvements in DC-based vaccination protocols remain of vital importance if large scale tumor eradication is to be achieved.

Dendritic cells are a heterogeneous group of cells in the body that differ in localization, phenotype and function. In the peripheral blood, the two main types are the CD11c- plasmacytoid DC (pDC) and CD11c+ myeloid DC (myDC). Although pDC comprise only 0.1% of all peripheral blood mononuclear cells (PBMC), they are crucial in antiviral immunity through the production of large quantities of type I interferons (6; 7). myDC can be further subdivided into cells that differentially express BDCA1 (CD1c), CD16, and BDCA3 (8; 9). Although CD16+-myDC comprise 65-75% of the total myDC population, BDCA1+-myDC (10-20%) are best described in literature and are thought to be the most potent T cell stimulators of the three myDC subpopulations (8).

In vivo, monocytes are capable of differentiating into myDC after sensing an inflammatory signal, maintaining a stable number of DC in the blood (7; 10). Ex vivo, monocyte-derived immature DC (iDC) are obtained by culturing monocytes in the presence of different growth factors (10).

Depending on the factors added, monocytes differentiate into DC with different functions and phenotypes (reviewed in (10)). Due to the low number of circulating DC in the blood, most DC-based vaccine studies use these ex vivo cultured iDC. The current standard cocktail for inducing monocyte activation and differentiation towards iDC contains GM-CSF and IL-4.

As professional antigen presenting cells (APC), DC have a central role in activating the innate and adaptive immune system and are able to tailor the immune response to the specific nature of a pathogen. iDC take up pathogens via endocytosis, process it, and present small peptide fragments (antigens) of that specific pathogen on their cell surface through MHC class I and class II molecules.

Upon pathogen recognition, DC undergo maturation. This process involves downregulating their phagocytic ability, while upregulating production of inflammatory cytokines and expression of surface molecules, such as co-stimulatory molecules and chemokine receptors (11). The nature of the pathogen dictates which cytokines and surface molecules are expressed by the DC. This process is mediated by pattern recognition receptors (PRR) and allows the DC to initiate an immune response tailored to the specific pathogen. The migration of mature DC (mDC) towards the lymph nodes contributes to their ability to mount immune responses, because the architecture of these organs is such that encounters of mDC with adaptive immune effector cells are maximized (12).

In tumor immunity, it is important that DC mount a potent TH1-polarized response to maximize the antigen-specific tumor killing by CTL (13). More recently, the importance of recruiting and activating NK cells has come to light, as they are not only capable of lysing tumor cells, but also help propagate the TH1-response (21).

NK cells are part of the innate immune system and play an important role in the immune surveillance of transformed cells, as they provide a first line of defense for virally transformed and malignant cells (14). The activation of NK cells is regulated by a delicate balance between activating and inhibiting receptors. In healthy cells, the balance favors inhibition, thereby protecting the cell from lysis (15). The activation of NK cells occurs either by the loss of inhibitory signals or by the gain of activating signals, providing two distinct mechanisms through which NK cells can perform their function (15). Inhibitive signals are provided to NK cells by the recognition of MHC class I molecules by Killer-cell immunoglobulin-like receptors (KIRs), and of non-classical HLA-E by NKG2A (16).

Each KIR recognizes a specific HLA-molecule and NK cells are known to be heterogeneous in their KIR expression (16). This specificity and distribution allow for continual survey of each HLA-molecule by NK cells. Both virally infected and malignant cells are known to downregulate MHC class I molecules to escape detection by the adaptive immune system (17). This downregulation results in the loss of inhibitory signals in NK cells, which become activated and lyse the cell. The activation of NK cells through loss of inhibition is referred to as "missing-self-recognition". Receptors that are involved in relaying activating signals include natural cytotoxicity receptors (e.g. NKp30, NKp40 and NKp46), NKG2D, and 2B4. Functional studies indicate that malignancies often express ligands to these receptors (16). The presence of these ligands could stimulate the receptors to a degree that the balance will tip towards activation and overrule and inhibitory signals provided by MHC-molecules, thereby activating NK cells. This pathway of NK cell activation is known as "induced-self-recognition".

In addition to their immune surveillant function for transformed cells, NK cells also have an immunoregulatory function by producing cytokines, such as TNF-alpha and IFN-gamma. This function of NK cells is especially important in NK-DC crosstalk. This crosstalk results in the reciprocal enhancement of functions, and has received increasing interest as an important factor in anti-tumor immunity. On the one hand DC activate NK cells by secreting cytokines, such as IL-12, IL-18, and IL-15 (with IL-2). IL-15 was shown to enhance NK cell survival (18), while IL-12 promoted NK cell IFN-gamma production (19; 20). Additionally, DC can recruit NK cells to the secondary lymph nodes in a CCR5 dependent manner (21), where NK cells provide an early source of IFN-gamma that aids TH1-polarization and CTL priming by DC (22; 23). These effects of NK-DC crosstalk on T cells are crucial in anti-tumor immunity, as they prime TH1 cells and CTL to eradicate the tumor in an antigen-specific manner (24). Subsequently, trials using DC that activate NK cells show an enhanced anti-tumor activity (25; 26).

On the other hand, NK cells are able to kill immunosuppressive iDC, as well as inappropriately matured DC, thereby ensuring the quality of the immune response (27). Under certain circumstances, NK cells are also capable of maturing DC by the secretion of TNF-alpha and IFN-gamma (20). These DC express high levels of CCR7, thereby facilitating migration to the secondary lymph nodes (20). Through these different interactions with DC, NK cells are capable of modulating the adaptive immune response. NK-DC crosstalk is mainly thought to take place in abused tissue and secondary lymph nodes, with responses differing according to the site of encounter (28).

DC and other cells of the innate immune system, such as NK cells, need to be able to directly identify specific pathogens in order to activate the appropriate immune response (31). They are capable of doing this via pattern recognition receptors (PRR), which recognize highly conserved pathogen associated molecular patterns (PAMP), typical for pathogens and that are never present in a healthy host. Depending on the PAMP encountered, different PRR and their signaling pathways become activated.

Cells differ in their expression of PRR, making them differentially susceptible to pathogens. Due to their central role in the immune system, DC express a wide array of PRR, including Toll-like receptors (TLR), nucleotide-binding oligomerization domain (NOD)-like receptors, C-type lectin receptors (CLR) and retinoic acid-inducible gene 1 (RIG-1)-like receptors. These receptors recognize different classes of pathogens, which ensures the induction of the appropriate immune response (30).

The best described PRR are members of the Toll-like receptor family, which can be found both intra- and extracellularly. The intracellular TLR 3, TLR 7, TLR 8, and TLR 9 are located in endosomes and recognize bacterial and viral DNA or RNA. The extracellular TLR 1, TLR 2, TLR 4, TLR 5 and TLR 6 are located on the cell surface and recognize membrane components of pathogens, such as LPS, peptidoglycan, and flagellin. All the TLR, except TLR3, signal in a Myd88-dependent manner, leading to NF-kappa B activation. In addition, TLR3 and TLR4 activate NF-kappa B and other transcription factors important for type I IFN production, via TRIF (31).

CLR can be found on the cell-membrane and are part of the fungal recognizing mechanism of the immune system, ultimately activating NF-kappa B. They also play an important role in antigen-uptake and presentation. More recently they have been shown to be important regulators of DC migration (32).

NOD- and RIG-like receptors are found in the cytoplasm, recognizing viral and bacterial components that are present in the cell. Their activation triggers the production of type I IFN through NF-kappa B (33; 34).

Depending on the PAMP encountered, different PRR and their signaling pathways become activated. One pathogen typically activates more than one PRR. Depending on the PRR combination triggered, a distinct signaling cascade is initiated that dictates the immune response and maturation program of the DC. This maturation program regulates the expression of co-stimulatory molecules, pro-inflammatory cytokines, chemokines and their receptors (30). The fact that different DC subsets vary in their adequacy to respond to different triggers can be explained by the fact that they are not equal in their expression of PRR (reviewed in 7). It is thought that triggering specific combinations of PRR will lead to the maturation of DC most suitable for anti-cancer vaccines, leading to potent TH1-skewing and NK cell responses. However, it is unknown which combination of PRR is optimal for inducing this effect.

Research has focused on identifying PRR-triggers that are capable of producing a mature DC phenotype with a predominant IL-12 cytokine profile that can enhance CTL activation and NK cell responses by providing a TH1-response. It has been established that the addition of membrane fragments of *Klebsiella pneumoniae* (FMKp; TLR2/4-trigger) together with IFN-gamma to the DC maturation cocktail, produces DC that are superior to the "golden standard" which are DC matured in the presence of a TNFalpha/PGE2-cocktail. The FMKp/IFN-gamma cocktail has proven to be in vitro superior in regard to CTL activation (35), IL-12 secretion and subsequent TH1-polarization (36). FMKp/IFN-gamma-matured DC were shown to produce more chemokines capable of attracting other immune cells (35).

As a representative example, we mention here dendritic cells which are transiently transfected with mRNA encoding the tumor antigen WT-1, a common, tumor specific transcription factor not expressed by somatic cells. The construct has been codon optimized and contains a specific targeting sequence resulting in both MHC-I and MHC-II presentation of WT-1 specific peptides to both CD8+ and CD4+ T cells in the patients. This initiates a strong, tumor specific immune response after reintroduction into the patient.

Despite these promising results, there is still room for improvement. The present invention provides an improved method for the production of mature dendritic cells *in vitro* using a bacterial lysate.

### Summary of the invention

The invention relates to a method for the production of mature dendritic cells in vitro or ex vivo, wherein immature dendritic cells are contacted with a composition comprising growth factors and a bacterial lysate, characterized in that the bacterial lysate is obtained by a method comprising the steps of providing a suspension comprising bacteria, sonicating the suspension followed by heat treatment of the suspension and optionally isolating mature dendritic cells from the composition.

The invention also relates to a method for the preparation of a bacterial lysate, comprising the steps of a) providing a suspension comprising bacteria, b) sonicating the suspension followed by c) heat treatment of the suspension, thereby obtaining a bacterial lysate capable of increasing the production of IL-12 by dendritic cells when contacted in vitro with said dendritic cells, wherein the increase of the production of IL-12 is higher than the increase when the lysate is prepared by a method wherein the bacterial suspension in step a) above is first subjected to heat treatment followed by a sonication step.

The invention also relates to a bacterial lysate obtainable by a method according to any one of claims 7 - 12, capable of increasing the production of IL-12 by dendritic cells when contacted in vitro with said dendritic cells, wherein the increase of the production of IL-12 is higher than the increase when the lysate is prepared by a method wherein the bacterial suspension is first heat-treated followed by a sonication step.

### Detailed description of the invention

The invention is concerned with a method for the improved production of dendritic cells and compositions useful in such a method. The model system that we employed was as follows. At day 0, PBMC were isolated from fresh blood, followed by isolation of CD14 positive cells (monocytes) from these PBMCs using CD14 beads and CliniMACS columns. CD14 positive cells were put into culture and differentiated into immature DC (iDC) using appropriate cytokines, in particular IL-4 and GM-CSF. At day 3, the culture medium was refreshed by performing a half medium exchange using differentiation medium containing IL-4 and GM-CSF. At day 6, iDC were matured into mDC by incubating them for 4 hours at 37°C and 5% CO2 using different maturation cocktails containing IL-4, GM-CSF, IFN-y and CL075. To this mixture also bacterial membrane fragments were added, in particular membrane fragments obtained from Klebsiella pneumoniae (KpMF).

Immediately after maturation, mDC were transfected with eGFP mRNA using the BioRad Gene pulser electroporation system. After maturation and electroporation, a cell count was performed using Via-1 cassettes and NucleoCounter followed by phenotype determination of mDC by FACS staining (CD80, CD83, CD86, HLA-DR and CD11c). 2.10⁶ DC were transferred to a new 24-wells plate and incubated for 16 hours at 37°C and 5% CO2. Then, the production of IL-10 and IL-12 was determined in the supernatant by Cytometric Bead Array while mDC were checked for eGFP expression by FACS.

A maturation cocktail successfully used to stimulate iDC to develop into mature DCs comprises IL-4, GM-CSF in combination with IFN-gamma, CL075 and a bacterial lysate termed KpMF (EP 2847321 B1). Such a bacterial lysate is commonly produced by sonication of isolated bacterial cells thereby lysing the cells and obtaining a lysate comprising membrane fragments. We now found that the bacterial lysate can even be further improved if it is prepared in a different way than so far described.

The present document describes a way to make a novel bacterial lysate that can successfully be used to further improve the maturation of immature dendritic cells (iDC). Traditionally, KpMF is prepared in a process wherein a bacterial suspension is subjected to sonication (51), sometimes preceded by heat treatment such as a process described in US Patent 7,033,591 B1.

We prepared four different batches of bacterial membrane fragments (Table 1). One batch (preparation #1) was only sonicated according to the prior art disclosed in reference 51, a second one was only heat treated, a third was first heat treated, followed by sonication (according to US patent 7,033,591 B1) and a fourth batch was first sonicated, following by heat treatment. The experimental details are described herein further.

**Table 1: Four different compositions comprising membrane fragments**

| **Sample #** | **Treatment** |
|---|---|
| 1 | Sonication |
| 2 | Heat treatment |
| 3 | First heat treatment, followed by sonication |
| 4 | First sonication, followed by heat treatment. |

These 4 samples were used in 4 different maturation cocktails. The data showed that all cells were DC, being highly positive for CD11c and HLA-DR. Maturation of DC is shown by 2-3 times upregulation of the median value for costimulatory markers CD80, CD83 and CD86. There were no phenotypical differences observed between the samples prepared with the different membrane fragment samples #1 - #4.

Four preparations of mDCs were prepared, using KpMF that was subjected to sonication alone (preparation #1) to heat treatment alone (preparation #2), to a process of first heat treatment followed by sonication (preparation #3) and a preparation that was first subjected to sonication followed by heat treatment (preparation #4). Immature DCs that were not subjected to the maturation cocktail served as the negative control.

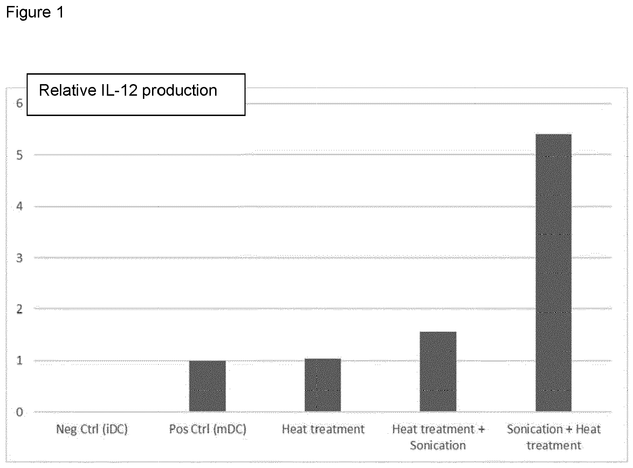

Surprisingly, we found that the membrane preparation #4, which was first sonicated followed by a heat inactivation step, yielded a 3 times higher IL-12 production as compared to the prior art preparation #3 (heat inactivation followed by sonication) and was therefore a much more potent maturation inducer (figure 1). We also observed that the use of a maturation cocktail comprising preparation #4 yielded a 5 times higher IL-12 production as compared to a process wherein a maturation cocktail according to the prior art (preparation #1, positive control in figure 1) was used..

The invention therefore relates to a method for the production of mature dendritic cells *in vitro* or ex *vivo,* wherein immature dendritic cells are contacted with a composition comprising growth factors and a bacterial lysate, characterized in that the bacterial lysate is obtained by a method comprising the steps of:
a) providing a suspension comprising bacteria,
b) sonicating the suspension followed by
c) heat treatment of the suspension.
Mature dendritic cells may then optionally be isolated from the composition.

The skilled person is well aware of the kind of growth factors that may be used for the maturation of immature DC into mature DC. In a particularly advantageous embodiment of the invention growth factors are selected from the group consisting of interferon-gamma (IFN-gamma), the pattern recognition receptor-ligand (PRR-ligand) CL075 and the PRR-ligand poly I:C.

When the experiments were repeated with bacterial lysates obtained from commonly isolated Gram-negative organisms including Pseudomonas, Klebsiella, Proteus, Salmonella, Providencia, Escherichia, Morganella, Aeromonas, and Citrobacter, essentially the same results were obtained. We conclude that the source and kind of bacteria is not critical and that all kinds of bacteria may be used to produce the membrane fragments as described herein. In a particularly advantageous embodiment, the bacteria are Gram-negative bacteria or bacteria containing endotoxin or OmpA, which are potential triggers to Toll like receptors. Particularly preferred are bacteria from the family of Enterobacteriaceae, even more preferably from the genus Klebsiella, such as Klebsiella pneumoniae.

The heat treatment step is intended to sterilize the composition. That may be achieved by conventional ways, well known in the art. For practical reasons, the heat treatment includes heating the composition to a temperature above 100 degrees Celsius for an appropriate amount of time. The skilled person is well aware of methods suitable for determining whether a composition is sterile, for instance by plating the composition on a rich medium agar plate. Particularly preferred are even higher temperatures because they allow the procedure to be much quicker, such as temperatures above 110 degrees Celsius. Even more preferred are again higher temperatures, such as above 120 degrees Celsius, such as 121 degrees Celsius. Therefore, in a preferred embodiment, the invention relates to a method for the production of a suspension as described herein, which is sterile. Heat treatment also includes autoclaving.

The term "autoclaving" is used herein to indicate a process wherein a composition is heated to a certain temperature (typically 121 degrees Celsius) for a certain amount of time (typically 15 minutes) in order to obtain a sterile composition. This process is usually performed under elevated pressure, i.e. above 1 bar.

The invention also relates to a method for the preparation of a bacterial lysate, comprising the steps of a) providing a suspension comprising bacteria, b) sonicating the suspension followed by c) heat treatment of the suspension, thereby obtaining a bacterial lysate capable of increasing the production of IL-12 by dendritic cells when the suspension is contacted in vitro with said dendritic cells, wherein the increase of the production of IL-12 is higher than the increase when the lysate is prepared by a method wherein the bacterial suspension in step a) above is first subjected to heat treatment followed by a sonication step.

For the same reasons as described above, the invention also relates to such a method wherein the growth factors are selected from the group consisting of interferon-gamma (IFN-gamma, the pattern recognition receptor-ligand (PRR-ligand) CL075 and the PRR-ligand poly I:C. The bacteria are advantageously Gram-negative bacteria or bacteria containing endotoxin or OmpA. Even more preferred, the bacteria are from the family of Enterobacteriaceae preferably from the genus Klebsiella, such as Klebsiella pneumoniae.

The term "sonication" or "sonicated" is used herein to indicate a process wherein a liquid, suspension or composition is exposed or subjected to high frequency sound waves, also known as ultrasound, in such a way that the components of the liquid, suspension or composition physically disintegrate or are disrupted.

The heat treatment may include a treatment at a temperature above 100 degrees Celsius, such as above 110 degrees Celsius, such as above 120 degrees Celsius, such as 121 degrees Celsius. This treatment preferably results in a sterile suspension.

In a further embodiment, the invention relates to a bacterial lysate obtainable by a method as described herein, capable of increasing the production of IL-12 by dendritic cells when contacted in vitro with said dendritic cells, wherein the increase of the production of IL-12 is higher than the increase when the lysate is prepared by a method wherein the bacterial suspension is first heat-treated followed by a sonication step.

### Legend to the figure

Mean of normalized values of IL-2 production by maturating DCs in the presence of 5 different maturation cocktails comprising 5 different preparations of bacterial membrane fragments (KpMF).

### Examples

### Example 1: Experimental procedures.

### Preparation of bacterial lysate

A stock *Klebsiella pneumoniae* vial is thawed and a droplet from the *Klebsiella pneumoniae* stock is added to 6 mL Nutrient Broth using an inoculation loop. This culture is incubated for 7 hours at 35 ± 2 °C on a rocking platform. Two mL bacterial suspension is added to 150 mL Nutrient Broth and incubated shaking for at least 16 hours at 35 ± 2 °C. The bacterial suspension is pelleted by centrifugation at 2000 g for 15 minutes at 20 ± 3 °C in 50 ml tubes. After discarding the supernatant by decanting, the cell pellets are resuspended in 50 mL PBS by vortexing and pipetting up and down. This step is repeated 3 times. Finally, the pellets are resuspended in 750 µL PBS and combined in one 15 mL tube. Again, the suspension is aliquoted as 750 µL bacterial suspension in Eppendorf tubes, followed by pelleting the suspension by centrifugation at 14000 g for 15 minutes at RT. Pellets were frozen at -80 °C ± 10 °C and kept for at least 4 hours.

### Sonication

Each cell pellet was thawed at room temperature and resuspended in 2.0 ml sonication buffer (5 mM Na₂EDTA / pH 7.0 ± 0.3) in a 15 ml tube. The tube was placed in a lab cooler during sonication. The sonotrode (Hielscher Ultrasonics Sonotrode S26D7 Titanium 7mm) was set at Amplitude 100% (180 µm) and Pulse control mode 0.9 and attached to an Ultrasonic Processor UP200Ht. The solution was sonicated on ice for at least 2 times, more preferably 5, 6, 7, 8 ,9 times or even more preferably 10 times, each time with 10 pulses more preferably 15 or 20 pulses and even more preferably 25 pulses. Each time for at least 10 seconds, or 20 seconds or more preferably 30 seconds. Also, each pulse may last 10 minutes, more preferably 5 minutes or even more preferably for 1 minute.

The solution was then centrifuged for 15 minutes at 14000 RCF in sterilized micro centrifuge tubes. Afterwards the supernatant was collected for further steps. A sample was taken of the collected supernatant to measure the concentration at A280 using a spectrophotometer.

The final concentration of the supernatant was adjusted with phosphate buffered saline (PBS) to the desired concentration based on the A280 result where ABS280 of 1 corresponds to 1 mg /mL cell membrane lysate (containing sugars, proteins and lipids). Typically, the A280 after sonication of a sample was between 1 and 1,5 AU which corresponds to 20 - 30 mg/ml lysate. The A280 of the final membrane product was typically between 0,7 and 0,85 AU, which corresponds to 3,5 - 4,5 mg/ml lysate after a dilution of 5 times.

### Sterilization

Filled Sterilized Crimp Vials 1.5 mL (VWR Collection) from each lysate were sterilized in an autoclave. Program for 2L liquid material was used (121 °C for 15 min). Vials were placed in a tray so they are kept upright during the run. Vials were labelled after sterilization and stored at -80 °C.

### Example 2: Process for the preparation of mature dendritic cells (mDC)

At day 0, PBMC were isolated from fresh blood, followed by isolation of CD14 positive cells (monocytes) from these PBMCs using CD14 beads and CliniMACS columns. CD14 positive cells were put into culture and differentiated into immature DC (iDC) using IL-4 and GM-CSF. At day 3, the culture medium was refreshed by performing a half medium exchange using differentiation medium containing IL-4 and GM-CSF. At day 6, iDC were matured into mDC by incubating them for 4 hours at 37°C and 5% CO2 using different maturation cocktails containing IL-4, GM-CSF, IFN-gamma, CL075 and 3 different stocks of KpMF. Immediately after maturation, mDC were transfected with eGFP mRNA using the BioRad Gene pulser electroporation system. After maturation and electroporation, a cell count was performed using Via-1 cassettes and NucleoCounter followed by phenotype determination of mDC by FACS staining (CD80, CD83, CD86, HLA-DR and CD11c). 2E6 DC were transferred to a new 24-wells plate and incubated for 16 hours at 37°C and 5% CO2. Then, the production of IL-12 was determined in the supernatant by Cytometric Bead Array while mDC were checked for eGFP expression by FACS.

### Legend to the figure.

Figure 1: The figure shows the normalized IL-12 production by mature DC, 20 hours after induction of maturation with different preparations of membrane fragments (table 1). Monocytes were positively selected by CD14 from peripheral blood as described. Cells were subsequently cultured for 6 days in GM-CSF and IL-4 to obtained differentiated immature DCs. One sample was kept as immature DC by no addition of maturation cocktail (negative control). Mature dendritic cells were obtained by adding a maturation cocktail of IFN-gamma and CL075 at proper concentrations per 2E6 DC/well. Different preparations of membrane fragments were finally added to this mixture and DC were allowed to mature for 4 hours after which the cells were washed and allowed to further mature for 20 hours. A previous preparation of membrane fragments (KpMF), that only had been sonicated, was used as a positive control. Supernatant was taken from all cultures and 50 microliter per sample was subjected to Cytometric Bead Array analysis specifically for IL-12 on a FACS cytometer.

### References

1. WHO: http://www.who.int/mediacentre/factsheets/fs297/en/ Accessed April 23, 2012.
2. Yang L, Carbone DP. Tumor-host immune interactions and dendritic cell dysfunction. Advances in Cancer Research. 2004 Jan; 9213-27.
3. Davis ID. Rational approaches to human cancer immunotherapy. Journal of Leukocyte Biology. 2003 Jan 1;73(1):3-29.
4. Dougan M, Dranoff G. Immune therapy for cancer. [Internet]. Annual review of Immunology. 2009 Jan 20;2783-117.
5. Ridgway D. The First 1000 Dendritic Cell Vaccinees. Cancer Invest. 2003;21(6):873-86.
6. Colonna M, Trinchieri G, Liu Y-J. Plasmacytoid dendritic cells in immunity. Nature Immunology. 2004 Dec 17;5(12):1219-26.
7. Schreibelt G, Tel J, Sliepen KHEWJ, Benitez-Ribas D, Figdor CG, Adema GJ, et al. Toll-like receptor expression and function in human dendritic cell subsets: implications for dendritic cell-based anti-cancer immunotherapy. Cancer Immunology, Immunotherapy. 2010 ;59(10):1573-1582.
8. MacDonald KPA, Munster DJ, Clark GJ, Dzionek A, Schmitz J, Hart DNJ. Characterization of human blood dendritic cell subsets. Blood. 2002 Dec 15;100(13):4512-20.
9. Piccioli D, Tavarini S, Borgogni E, Steri V, Nuti S, Sammicheli C, et al. Functional specialization of human circulating CD16 and CD1c myeloid dendritic-cell subsets. Blood. 2007 Jun 15;109(12):5371-9.
10. Banchereau J, Palucka AK. Dendritic cells as therapeutic vaccines against cancer. Nature Reviews Immunology. 2005 Apr 1;5(4):296-306.
11. Banchereau J, Briere F, Caux C, Davoust J, Lebecque S, Liu YJ, et al. Immunobiology of dendritic cells. Annual Review of Immunology. 2000 Jan 28;18767-811.
12. Figdor CG, Vries IJM de, Lesterhuis WJ, Melief CJM. Dendritic cell immunotherapy: mapping the way. Nature Medicine. 2004 May 30;10(5):475-80.
13. Kalinski P, Okada H. Polarized dendritic cells as cancer vaccines: directing effector-type T cells to tumors. [Internet]. Seminars in immunology. 2010 Jun ;22(3):173-82.
14. Mailliard RB, Alber SM, Shen H, Watkins SC, Kirkwood JM, Herberman RB, et al. IL-18-induced CD83+CCR7+ NK helper cells. Journal of Experimental Medicine. 2005 Oct 3;202(7):941-53.
15. Raulet DH, Vance RE. Self-tolerance of natural killer cells. Nature Reviews Immunology. 2006 Jul 1;6(7):520-31.
16. Degli-Esposti MA, Smyth MJ. Close encounters of different kinds: dendritic cells and NK cells take centre stage. Nature Reviews Immunology. 2005 Feb 1;5(2):112-24.
17. Vasievich EA, Huang L. The suppressive tumor microenvironment: a challenge in cancer immunotherapy. Molecular Pharmaceutics. 2011 Jun 6;8(3):635-41.
18. Cooper MA, Bush JE, Fehniger TA, VanDeusen JB, Waite RE, Liu Y, et al. In vivo evidence for a dependence on interleukin 15 for survival of natural killer cells. Blood. 2002 Nov 15;100(10):3633-8.
19. Ferlazzo G, Pack M, Thomas D, Paludan C, Schmid D, Strowig T, et al. Distinct roles of IL-12 and IL-15 in human natural killer cell activation by dendritic cells from secondary lymphoid organs. Proceedings of the National Academy of Sciences USA. 2004 Nov 23;101(47):16606-11.
20. Vitale M, Della Chiesa M, Carlomagno S, Pende D, Aricò M, Moretta L, et al. NK-dependent DC maturation is mediated by TNFalpha and IFNgamma released upon engagement of the NKp30 triggering receptor. Blood. 2005 Jul 15;106(2):566-71.
21. Van Elssen CHMJ, Vanderlocht J, Frings PWH, Senden-Gijsbers BLMG, Schnijderberg MCA, Gelder M van, et al. Klebsiella pneumoniae-triggered DC recruit human NK cells in a CCR5-dependent manner leading to increased CCL19-responsiveness and activation of NK cells. European Journal of Immunology. 2010 Nov ;40(11):3138-49.
22. Mailliard RB, Son Y-I, Redlinger R, Coates PT, Giermasz A, Morel PA, et al. Dendritic Cells Mediate NK Cell Help for Th1 and CTL Responses: Two-Signal Requirement for the Induction of NK Cell Helper Function. J. Immunol. 2003 ;171(5):2366-2373.
23. Martin-Fontecha A, Thomsen LL, Brett S, Gerard C, Lipp M, Lanzavecchia A, et al. Induced recruitment of NK cells to lymph nodes provides IFN-gamma for T(H)1 priming. Nature Immunology. 2004 Dec 7;5(12):1260-5.
24. Kalinski P, Urban J, Narang R, Berk E, Wieckowski E, Muthuswamy R. Dendritic cell-based therapeutic cancer vaccines: what we have and what we need. Future Oncology. 2009 Apr 1;5(3):379-90.
25. Karimi K, Boudreau JE, Fraser K, Liu H, Delanghe J, Gauldie J, et al. Enhanced antitumor immunity elicited by dendritic cell vaccines is a result of their ability to engage both CTL and IFN gamma-producing NK cells. Molecular Therapy:. 2008 Feb 4;16(2):411-8.
26. Fernandez NC, Lozier A, Flament C, Ricciardi-Castagnoli P, Bellet D, Suter M, et al. Dendritic cells directly trigger NK cell functions: cross-talk relevant in innate antitumor immune responses in vivo. Nature Medicine. 1999 Apr 1;5(4):405-11.
27. Ferlazzo G. Human Dendritic Cells Activate Resting Natural Killer (NK) Cells and Are Recognized via the NKp30 Receptor by Activated NK Cells. Journal of Experimental Medicine. 2002 Feb 4;195(3):343-351.
28. Moretta A. Natural killer cells and dendritic cells: rendezvous in abused tissues. Nature Reviews Immunology. 2002 Dec 1;2(12):957-64.
29. Muzio M, Bosisio D, Polentarutti N, D'amico G, Stoppacciaro A, Mancinelli R, et al. Differential Expression and Regulation of Toll-Like Receptors (TLR) in Human Leukocytes: Selective Expression of TLR3 in Dendritic Cells. J. Immunol. 2000 ;164(11):5998-6004.
30. Padovan E, Landmann RM, De Libero G. How pattern recognition receptor triggering influences T cell responses: a new look into the system. Trends in Immunology. 2007 Jul ;28(7):308-14.
31. Lee MS, Kim Y-J. Signaling pathways downstream of pattern-recognition receptors and their cross talk. Annual review of biochemistry. 2007 Jan 19;76447-80.
32. Figdor CG, Kooyk Y van, Adema GJ. C-type lectin receptors on dendritic cells and Langerhans cells. Nature Reviews Immunology. 2002 Feb 1;2(2):77-84.
33. Fritz JH, Girardin SE. How Toll-like receptors and Nod-like receptors contribute to innate immunity in mammals. Journal of Endotoxin Research. 2005 Dec 1;11(6):390-394.
34. Cella M. Maturation, Activation, and Protection of Dendritic Cells Induced by Doublestranded RNA. Journal of Experimental Medicine. 1999 Mar 1;189(5):821-829.
35. Vanderlocht J, Elssen CHMJ van, Senden-Gijsbers BLMG, Meek B, Cloosen S, Libon C, et al. Increased tumor-specific CD8+ T cell induction by dendritic cells matured with a clinical grade TLR-agonist in combination with IFN-g. International Journal of Immunopathology and Pharmacology. 2010 ;23(1):35-50.
36. Oth T. Role of selective pattern recognition receptor-triggering in dendritic cell-mediated type 1 response. Internal Medicine, Haematology. 2010; Master Thesis 56.
37. Wang D, Dubois RN. Eicosanoids and cancer. Nature Reviews Cancer. 2010 Mar 19;10(3):181-93.
38. Boniface K, Bak-Jensen KS, Li Y, Blumenschein WM, McGeachy MJ, McClanahan TK, et al. Prostaglandin E2 regulates Th17 cell differentiation and function through cyclic AMP and EP2/EP4 receptor signaling. The Journal of Experimental Medicine. 2009 Mar 16;206(3):535-48.
39. Snijdewint FG, Kaliński P, Wierenga EA, Bos JD, Kapsenberg ML. Prostaglandin E2 differentially modulates cytokine secretion profiles of human T helper lymphocytes. Journal of Immunology. 1993 Jun 15;150(12):5321-9.
40. Zeddou M, Greimers R, Valensart N de, Nayjib B, Tasken K, Boniver J, et al. Prostaglandin E2 induces the expression of functional inhibitory CD94/NKG2A receptors in human CD8+ T lymphocytes by a cAMP-dependent protein kinase A type I pathway. Biochemical Pharmacology. 2005 Sep 1;70(5):714-24.
41. Ahmadi M, Emery DC, Morgan DJ. Prevention of both direct and cross-priming of antitumor CD8+ T-cell responses following overproduction of prostaglandin E2 by tumor cells in vivo. Cancer Research. 2008 Sep 15;68(18):7520-9.
42. Walker W, Rotondo D. Prostaglandin E2 is a potent regulator of interleukin-12- and interleukin-18-induced natural killer cell interferon-gamma synthesis. Immunology. 2004 Mar ;111(3):298-305.
43. Yakar I, Melamed R, Shakhar G, Shakhar K, Rosenne E, Abudarham N, et al. Prostaglandin e(2) suppresses NK activity in vivo and promotes postoperative tumor metastasis in rats. Annals of Surgical Oncology. 2003 May ;10(4):469-79.
44. Verdijk P, Aarntzen EHJG, Lesterhuis WJ, Boullart ACI, Kok E, Rossum MM van, et al. Limited amounts of dendritic cells migrate into the T-cell area of lymph nodes but have high immune activating potential in melanoma patients. Clinical Cancer Research. 2009 Apr 1;15(7):2531-40.
45. Kalinski P, Schuitemaker JHN, Hilkens CMU, Wierenga EA, Kapsenberg ML. Final Maturation of Dendritic Cells Is Associated with Impaired Responsiveness to IFN-{gamma} and to Bacterial IL-12 Inducers: Decreased Ability of Mature Dendritic Cells to Produce IL-12 During the Interaction with Th Cells. J. Immunol. 1999 ;162(6):3231-3236.
46. Cools N, Van Tendeloo VFI, Smits ELJM, Lenjou M, Nijs G, Van Bockstaele DR, et al. Immunosuppression induced by immature dendritic cells is mediated by TGF-beta/IL-10 double-positive CD4+ regulatory T cells. Journal of cellular and molecular medicine. 2008 Apr ;12(2):690-700.
47. Spranger S, Javorovic M, Bürdek M, Wilde S, Mosetter B, Tippmer S, et al. Generation of Th1-polarizing dendritic cells using the TLR7/8 agonist CL075. Journal of immunology (Baltimore, Md. : 1950). 2010 Jul 1;185(1):738-47.
48. Boullart ACI, Aarntzen EHJG, Verdijk P, Jacobs JFM, Schuurhuis DH, Benitez-Ribas D, et al. Maturation of monocyte-derived dendritic cells with Toll-like receptor 3 and 7/8 ligands combined with prostaglandin E2 results in high interleukin-12 production and cell migration. Cancer immunology, immunotherapy : CII. 2008 Nov ;57(11):1589-97.
49. Nguyen KB, Salazar-Mather TP, Dalod MY, Van Deusen JB, Wei X-qing, Liew FY, et al. Coordinated and Distinct Roles for IFN-{alpha}{beta}, IL-12, and IL-15 Regulation of NK Cell Responses to Viral Infection. J. Immunol. 2002 Oct 15;169(8):4279-4287.
50. Mailliard RB, Wankowicz-Kalinska A, Cai Q, Wesa A, Hilkens CM, Kapsenberg ML, et al. alpha-type-1 polarized dendritic cells: a novel immunization tool with optimized CTL-inducing activity. Cancer research. 2004 Sep 1;64(17):5934-7.
51. Oth T, Habets THPM, Germeraad WTV, Zonneveld MI, Bos GMJ, Vanderlocht J. Pathogen recognition by NK cells amplifies the pro-inflammatory cytokine production of monocyte-derived DC via IFN-γ. BMC Immunol. 2018 Feb 13;19(1):8.
52. Oth T, Vanderlocht J, Van Elssen CH, Bos GMJ, Germeraad WTV. Pathogen-Associated Molecular Patterns Induced Crosstalk between Dendritic Cells, T Helper Cells, and Natural Killer Helper Cells Can Improve Dendritic Cell Vaccination. Mediat Inflammat: 2016;2016:5740373.
53. Oth T, Van Elssen CH, Schnijderberg MC, Senden-Gijsbers BL, Germeraad WTV, Bos GMJ, Vanderlocht J. Potency of Both Human Th1 and NK Helper Cell Activation is Determined by IL-12p70-Producing PAMP-Matured DCs. J Interferon Cytokine Res. 2015 Sep;35(9):748-58.
54. Oth T, Schnijderberg MC, Senden-Gijsbers BL, Germeraad WTV, Bos GMJ, Vanderlocht J. Monitoring the initiation and kinetics of human dendritic cell-induced polarization of autologous naive CD4+ T cells. PLoS One. 2014 Aug 21;9(8):e103725.
55. Van Elssen CHJM, Oth T, Germeraad WTV, Bos GMJ, Vanderlocht J. Natural killer cells: the secret weapon in dendritic cell vaccination strategies. Clin Cancer Res. 2014 Mar 1;20(5):1095-103. Review.
56. Van Elssen CHJM, Vanderlocht J, Oth T, Senden-Gijsbers BLMG, Germeraad WTV and Bos GMJ. Inflammation restraining effects of Prostaglandin E2 on DC-NK interaction are imprinted during DC maturation. Blood. 2011; 118 (9): 2473-2482.
57. Van Elssen CMJM, Vanderlocht J, Frings PWH, Senden-Gijsbers BLMG, Schnijderberg MCA, Gelder M van, Meek B, Libon C, Ferlazzo G, Germeraad WTV and Bos GMJ. Klebsiella pneumoniae triggered DCs recruit human NK cells in a CCR5-dependent manner leading to increased CCL19-responsiveness and activation of NK cells. Eur. J. Immunol. 2010. 40: 3138-3149.

## Claims

1. Method producing mature dendritic cells in vitro or ex vivo, wherein immature dendritic cells are contacted with a composition comprising growth factors and a bacterial lysate, **characterized in that** the bacterial lysate is obtainable by a method comprising the steps of:
a) providing a suspension comprising bacteria,
b) sonicating the suspension followed by
c) heat treatment of the suspension,
and optionally isolating mature dendritic cells from the composition.

2. Method according to claim 1 wherein the growth factors are selected from the group consisting of interferon gamma (IFN-gamma), the pattern recognition receptor-ligand (PRR-ligand) CL075 and the PRR-ligand poly I:C.

3. Method according to claim 1 or 2 wherein the bacteria are Gram-negative bacteria or bacteria containing endotoxin or OmpA.

4. Method according to claim 3 wherein the bacteria are selected from the group consisting of Pseudomonas, Klebsiella, Proteus, Salmonella, Providencia, Escherichia, Morganella, Aeromonas, and Citrobacter.

5. Method according to claim 3 wherein the bacteria are from the family of Enterobacteriaceae preferably from the genus Klebsiella, such as Klebsiella pneumoniae.

6. Method according to any one of claims 1 - 5 wherein the heat treatment includes a treatment at a temperature above 100 degrees Celsius, such as above 110 degrees Celsius, such as above 120 degrees Celsius, such as 121 degrees Celsius.

7. Method according to claim 6 wherein the heat treatment results in a sterile composition.

8. Method for preparing a bacterial lysate, comprising the steps of:
a) providing a suspension comprising bacteria,
b) sonicating the suspension followed by
c) heat treatment of the suspension,
thereby obtaining a bacterial lysate capable of increasing the production of IL-12 by dendritic cells when contacted *in vitro* with said dendritic cells, wherein the increase of the production of IL-12 is higher than the increase when the lysate is prepared by a method wherein the bacterial suspension in step a) is first subjected to heat treatment (step c)) followed by a sonication, step b).

9. Method according to claim 8 wherein the growth factors are selected from the group consisting of interferon gamma (IFN-gamma), the pattern recognition receptor-ligand (PRR-ligand) CL075 and the PRR-ligand poly I:C.

10. Method according to claim 8 or 9 wherein the bacteria are Gram-negative bacteria or bacteria containing endotoxin or OmpA.

11. Method according to claim 10 wherein the bacteria are selected from the group consisting of Pseudomonas, Klebsiella, Proteus, Salmonella, Providencia, Escherichia, Morganella, Aeromonas, and Citrobacter.

12. Method according to claim 10 wherein the bacteria are from the family of Enterobacteriaceae preferably from the genus Klebsiella, such as Klebsiella pneumoniae.

13. Method according to any one of claims 8 - 12 wherein the heat treatment includes a treatment at a temperature above 100 degrees Celsius, such as above 110 degrees Celsius, such as above 120 degrees Celsius, such as 121 degrees Celsius.

14. Method according to claim 13 wherein the heat treatment results in a sterile suspension.

15. Bacterial lysate obtainable by a method according to any one of claims 8 - 14, capable of increasing the production of IL-12 by dendritic cells when contacted *in vitro* with said dendritic cells, wherein the increase of the production of IL-12 is higher than the increase when the lysate is prepared by a method wherein the bacterial suspension is first heat-treated followed by a sonication step.
